# EUROPÄISCHE PATENTSCHRIFT

(11) **EP 1 309 363 B1**
(45) Veröffentlichungstag und Bekanntmachung des Hinweises auf die Patenterteilung: **14.02.2007**
(21) Anmeldenummer: 01943128.7
(22) Anmeldetag: 23.05.2001
(51) Int. Cl.: A61M 1/02, A61M 1/36, A61M 1/34

(54) **FILTERANORDNUNG ZUM AUFTRENNEN VON BLUT IN PLASMA UND ZELLULÄRE BESTANDTEILE SOWIE VORRICHTUNG FÜR DEREN EINSATZ AM SPENDER**
FILTER ARRANGEMENT FOR THE SEPARATION OF BLOOD INTO PLASMA AND CELLULAR COMPONENTS AND DEVICE FOR APPLICATION THEREOF ON THE DONOR
ENSEMBLE FILTRE POUR FRACTIONNER DU SANG EN PLASMA ET EN COMPOSANTS CELLULAIRES ET DISPOSITIF POUR SON APPLICATION SUR UN DONNEUR

(30) Priorität: 15.08.2000 DE 20014311 U
(43) Veröffentlichungstag der Anmeldung: 14.05.2003
(73) Patentinhaber: Heim Medizintechnik GmbH, 45966 Gladbeck (DE)
(72) Erfinder: HEIM, Gerd, H., 45964 Gladbeck (DE)
(74) Vertreter: Kreuzkamp, Markus
(86) Internationale Anmeldenummer: PCT/DE2001/001936
(87) Internationale Veröffentlichungsnummer: WO 2002/013888

(56) Entgegenhaltungen:
- EP-A- 0 414 515
- WO-A-97/32653
- DE-A- 19 733 407
- DE-U- 29 516 471

## Beschreibung

Die Erfindung betrifft eine Filteranordnung zum Auftrennen von Blut in Plasma und zelluläre Bestandteile gemäß den Merkmalen des Oberbegriffs des Patentanspruchs 1 sowie eine Vorrichtung für deren Einsatz am Spender gemäß den Merkmalen des Oberbegriffs des Patentanspruchs 12.

Die vorgenannten Filteranordnungen sind seit vielen Jahren Gegenstand intensiver Forschungs- und Entwicklungsbemühungen, um das bewährte, aber aufwendige Trennverfahren von Blut mittels einer Zentrifuge, das darüber hinaus wegen der dann erforderlichen Umfüllmaßnahmen auch stets die Gefahr einer Kontamination des Blutes mit Luft und damit auch mit Keimen in sich birgt, zur Herstellung von Eigen- oder Fremdblutkonserven durch ein einfaches Filtrationsverfahren ersetzen zu können, bei dem das Blut in einem sterilisierten geschlossenen System sowohl in seine einzelnen Komponenten, insbesondere Erythrozyten und Plasma, zerlegt als auch unmittelbar komponentenweise konserviert werden kann. Als Beispiele solcher Bemühungen seien hier nur die Gegenstände der DE 197 33 407 A1, der Veröffentlichung "Hämatologie, Bd. 1, Aktueller Stand der Eigenbluttransfusion, Sympomed-Verlag, München 1992, S. 108 bis 113" und der aus der internationalen Patentanmeldung PCT/DE 00/01258 hervorgegangenen Druckschrift WO 00/62840 angeführt.

Alle diese Bemühungen haben bisher jedoch in der Praxis nicht zu dauerhaft befriedigenden Ergebnissen hinsichtlich der möglichst weitgehenden eindeutigen Trennung von Plasma und zellulären Bestandteilen des jeweiligen Blutes geführt, d.h. nach der primären Filtration liegt der Hämatokritwert in der Fraktion der zellulären Bestandteile allenfalls in der Größenordnung von 50 %, was in keinem Falle den mit einer Zentrifuge erreichbaren Ergebnissen entspricht.

Die Filteranordnungen der vorbeschriebenen Art sind üblicherweise innerhalb einer Vorrichtung angeordnet, die aus mehreren Leitungen und Behältern für fluide Medien unterschiedlicher Viskosität sowie aus mindestens einem Anschlußelement, beispielsweise einer Kanüle, zur Ankopplung an den Blutkreislauf eines Spenders besteht, wobei sie außerdem noch weitere Filter bekannter Art, beispielsweise ein Leukozytendepletionsfilter, Rückschlagventile und/oder Einrichtungen zur Rückstauerzeugung, beispielsweise einstellbare Klemmvorrichtungen in Form von Rollklemmen, aufweisen kann. Wesentlich sind dabei - wie beispielsweise der vorgenannten Druckschrift WO 00/62840 entnehmbar ist - die gegenseitigen vertikalen Abstände der jeweiligen Filteranordnung und der zugehörigen vor- und nachgeschalteten Behälter, um die für einen optimalen Betrieb der jeweiligen Vorrichtung erforderlichen Druckdifferenzen zwischen Blutquelle und spezieller Filteranordnung einerseits sowie Filteranordnung und Behältern für Plasma und zelluläre Bestandteile andererseits an die jeweilige Art der Filteranordnung bestmöglich anzupassen und während des Einsatzes auch aufrecht zu erhalten. WO 00/62840 ist als nächstliegender Stand der Technik für die vorliegende Anmeldung anzusehen und zeigt eine Filteranordnung nach dem Oberbegriff von dem Anspruch 1.

Der vorliegenden Erfindung liegt daher die Aufgabe zugrunde, eine Filteranordnung zur Verfügung zu stellen, mit der auf einfache Weise auch in der Praxis hinreichend befriedigende Ausbeuten an den einzelnen Komponenten von Spenderblut erzielbar sind, um so den Bedarf an Blutkonserven sowohl in Form von Eigen- als auch von Fremdblut ohne großen Aufwand decken zu können, wobei die Filteranordnung in eine für einen optimalen Trennvorgang angepaßte Vorrichtung integrierbar ist.

Die vorliegende Erfindung löst diese Aufgabe mit Hilfe der Gesamtheit der Merkmale des Patentanspruchs 1 sowie der Gesamtheit der Merkmale des Patentanspruchs 12.

Dabei erweist es sich zunächst als besonders vorteilhaft, daß die feinporige Membran durch ein U-förmig ausgebildetes Bündel von n = 650 hydrophilen mikroporösen Polyethersulfan - Kapillarhohlfasern (Typ Micro PES-TF 10 der Akzo Faser AG) ausgebildet wird, deren Enden in einer gemeinsamen Lochplatte mit einem Eingangs- und einem Ausgangsbereich luft- und keimdicht fixiert sind und deren freie Länge zwischen dem Eingangs- und Ausgangsbereich der Lochplatte 1 = 27 cm beträgt, wobei die Lochplatte ihrerseits luft- und keimdicht in den offenen Endbereich eines nur einseitig offenen Gehäuses so eingepaßt ist, daß die freie Länge aller Hohlfasern innerhalb des durch die Lochplatte abgeschlossenen Gehäuses angeordnet ist und die Innenräume aller Hohlfasern die Einlaßkammer und der Außenraum aller Hohlfasern zwischen Lochplatte und dem geschlossenen Ende des Gehäuses die Auslaßkammer ausbilden, weil mit einer solchen Anordnung aufgrund umfangreicher Versuchsreihen bereits in einem einstufigen Verfahren stets ein Hämatokritwert von mindestens 62 % erreicht wird. Mit einem zweistufigen Verfahren, das beispielsweise durch eine Umkehr der Flußrichtung der separierten zellulären Bestandteile durch die Filteranordnung in einen vor deren Eingangsbereich zuschaltbaren zusätzlichen Sammelbehälter verwirklichbar ist, wird nicht nur stets der vorgenannte Hämatokritwert, sondern sogar stets ein Hämatokritwert von mindestens 80 % erreicht, wobei ein solcher von 90 % keine Seltenheit ist. Derartige Werte liegen deutlich über all denen, die bisher mit ein- oder auch mit zweistufigen Filtrationen erreicht wurden.

Die vorgenannte Druckschrift DE 197 33 407 A1 offenbart eine Filtereinrichtung zur Abtrennung von festen Bestandteilen aus Flüssigkeiten (z.B. Blut), wobei die Filtereinrichtung ein Hohlfaserfilter enthält, dessen Fasern aus Membranen Micro PES-TF 10 (Fa. Akzo) ausgebildet sind.

Die Druckschrift US 4,025,436 offenbart eine Filtereinrichtung mit einem Filterelement, das eine Einlaßkammer mit einer Auslaßkammer verbindet, als ein U-förmig Bündel aus Hohlfaser ausgebildet ist, und in einem mit zwei separaten Lochplatten abgeschlossenen Gehäuse angeordnet ist, wobei die Lochplatten die Enden von den Fasern luftdicht fixieren.

Vorteilhafte Weiterbildungen der in Rede stehenden Filteranordnungen werden durch die Gegenstände der Unteransprüche 2 bis 11 gegeben, wobei die Unteransprüche 2 bis 10 insbesondere vorteilhafte konstruktive Maßnahmen betreffen, die die Herstellung der erfindungsgemäßen Filteranordnung als luft- und keimdichtes geschlossenes System, das vor dem Gebrauch zwangsläufig als Ganzes sterilisiert werden muß, erheblich erleichtern und damit kostengünstig gestalten.

Der Unteranspruch 11 betrifft die vorteilhafte Anordnung einer Anschlußvorrichtung zur Einleitung von Kochsalzlösung in die erfindungsgemäße Filteranordnung, die dazu dient, die hydrophilen Hohlfasern der vorgenannten Art vor ihrem eigentlichen Trenn-Einsatz mit dieser Lösung zu spülen und damit die Trennwirkung der Hohlfasern von vornherein sicherzustellen.

Als sehr vorteilhaft erweist sich bei einer Vorrichtung für den Einsatz der vorgenannten Filteranordnung am Spender weiterhin, daß dem Eingangsbereich der Filteranordnung ein Sammelbehälter für das Spenderblut vor-, dem Auslaßbereich der Filteranordnung ein Aufnahmebehälter für zelluläre Bestandteile des Spenderblutes nach- und der Auslaßkammer der Filteranordnung ein Aufnahmebehälter für Blutplasma ebenfalls nachgeschaltet ist, wobei die Verbindungen zwischen der Filteranordnung und den vorgenannten Behältern über die Zuleitung bzw. die Ablaufleitungen luft- und keimdicht, aber mit Mitteln bekannter Art ohne Luftzutritt und Störeffekte durch unerwünschte Druckausgleichseffekte unterbrechbar ausgebildet sind, daß der Sammelbehälter mit vorgegebenem vertikalem Abstand seiner Unterkante oberhalb der Oberkante der Filteranordnung, der Aufnahmebehälter für Blutplasma mit vorgegebenem vertikalen Abstand seiner Oberkante unterhalb der Unterkante der Filteranordnung und der Aufnahmebehälter für zelluläre Bestandteile des Spenderblutes mit seiner Oberkante auf der Höhe der Unterkante der Filteranordnung angeordnet ist und daß der Sammelbehälter mit einer luft- und keimdicht mit ihm verbundenen Eingangsleitung verbunden ist, die an ihrem dem Sammelbehälter abgewandten Ende mit einem Anschlußelement zum Blutkreislauf des Spenders versehen ist, weil mit einer solchen Vorrichtung erreicht wird, daß das zu trennende Spenderblut nach seiner Sammlung an vorgegebenem Ort aufgrund der durch die Festlegung vorgegebener vertikaler Abstände zwischen Sammelbehälter und Filteranordnung einerseits sowie Filteranordnung und Aufnahmebehältern für zelluläre Bestandteile bzw. Blutplasma andererseits optimal vorgebbaren Druckverhältnisse in der vorbeschriebenen Filteranordnung tatsächlich in der bereits vorstehend angegebenen Größenordnung in seine Komponenten oder Gruppen zerlegt werden kann. Dieser Effekt wird vorteilhafterweise noch verstärkt; wenn darüber hinaus in der Zuleitung zwischen Sammelbehälter und Filteranordnung ein Leukozytendepletionsfilter bekannter Art angeordnet ist, da so die nachgeschaltete Filteranordnung weniger belastet wird und eine schnellere Auftrennung zwischen Blutplasma und verbliebenen zellulären Bestandteilen des Blutes ermöglicht - abgesehen davon, daß dadurch im Aufnahmebehälter für zelluläre Bestandteile im wesentlichen sofort ein üblicherweise gewünschtes weitgehend reines Erythrozyten-Konzentrat gewonnen wird.

Eine vorteilhafte Weiterbildung der vorliegenden Vorrichtung liegt auch vor, wenn in der Zuleitung zwischen Sammelbehälter und Filteranordnung eine Tropfkammer bekannter Art angeordnet ist, weil mit dieser Maßnahme das Angebot an zu trennendem Spenderblut für die Filteranordnung auf einfache Weise eingestellt und kontrolliert werden kann, wobei es sich bei gleichzeitiger Installation eines Leukozytendepletionsfilters vorteilhafterweise empfiehlt, die Tropfkammer zwischen Sammelbehälter und Leukozytendepletionsfilter anzuordnen, um einerseits auf einfache Weise die bereits vorerwähnte Einstellung und Kontrolle des Angebots an zu trennendem Spenderblut an die Filteranordnung einschließlich des Leukozytendepletionsfilters vornehmen zu können und andererseits einen ununterbrochenen gleichmäßigen Fluß des zu trennenden Spenderblutes sowohl durch den Leukozytendepletionsfilter als auch durch die Filteranordnung und damit auch konstante vorgegebene Druckverhältnisse in beiden Filtereinrichtungen zu gewährleisten, was eine der Voraussetzungen für den überraschenden Effekt eines vollkommem zellfreien Blutplasmas als Ergebnis des Einsatzes der in Rede stehenden Vorrichtung ist.

Bei einer anderen Ausführungsform der in Rede stehenden Vorrichtung erweist es sich auch als vorteilhaft, daß der vorgegebene vertikale Abstand zwischen der Unterkante des Sammelbehälters und der Oberkante der Filteranordnung a₁ = (100 ± 20) cm beträgt und/oder daß der vorgegebene vertikale Abstand zwischen der Unterkante der Filteranordnung und der Oberkante des Aufnahmebehälters für Blutplasma a₂ = 50 cm beträgt, da sich mit diesen Abstandswerten bei einschlägigen Versuchen optimale Werte der Komponententrennung ergeben haben.

Als vorteilhaft sind auch Ausführungsformen der erfindungsgemäßen Vorrichtung anzusehen, bei denen die Ablaufleitung der Einlaßkammer mit einer Einrichtung zur Erzeugung eines Rückstaus in der Filteranordnung versehen ist, insbesondere dann, wenn die Einrichtung zur Erzeugung eines Rückstaus eine einstellbare Klemmvorrichtung ist oder gar die einstellbare Klemmvorrichtung durch eine Rollklemme bekannter Art verwirklicht wird, weil sich mit einer solchen Einrichtung die grundsätzlich für alle Trennvorgänge einmal mittels der vertikalen Abstände zwischen Sammelbehälter und Filteranordnung einerseits sowie Filteranordnung und Aufnahmebehälter für zelluläre Bestandteile bzw. Blutplasma andererseits vorzugebenden Druckverhältnisse bei unvermeidlichen Schwankungen der Einstellungsparameter, beispielsweise durch Viskositätsänderungen des Blutes von einem Spender zum anderen oder Temperaturschwankungen von einem Trennvorgang zum anderen, auf einfache Art und Weise nachregeln lassen.

Vorteilhafte Weiterbildungen der in Rede stehenden Vorrichtung sind auch dann gegeben, wenn der Sammelbehälter mindestens im Startzeitpunkt der Blutaufnahme eine vorgegebene Menge einer Stabilisatorflüssigkeit bekannter Art enthält und/oder wenn der Aufnahmebehälter für zelluläre Bestandteile des Spenderblutes eine Vorfüllung mit einer vorgegebenen Menge einer Additivlösung bekannter Art aufweist, da damit auf bekannte Art und Weise sichergestellt wird, daß die erhaltenen Blutkomponenten oder Gruppen davon ausreichend lange konserviert werden können.

Als vorteilhaft ist auch eine Ausführungsform der vorliegenden Vorrichtung anzusehen, bei der das Anschlußelement eine Kanüle ist, weil letztere ein bewährtes und einsfach handhabbares Element zur Ankopplung einer Vorrichtung der in Rede stehenden Art an den Blutkreislauf eines Spenders darstellt.

Eine vorteilhafte Weiterbildung der gegebenen Vorrichtung liegt auch dann vor, wenn die Auslaßkammer über die Anschlußvorrichtung für Kochsalzlösung luft- und keimfrei mit einem Vorratsbehälter für eine solche Kochsalzlösung verbunden ist, weil dies eine einfache Methode darstellt, um die Hohlfasern zwangsläufig vor ihrem Einsatz mit physiologischer Kochsalzlösung in ausreichendem Maße zu spülen, um ihre Wirksamkeit für die Komponententrennung von vornherein zu optimieren.

Weiterhin ist es als vorteilhaft anzusehen, wenn bei der vorliegenden Vorrichtung die Ablaufleitung der Einlaßkammer mittels einer Entlüftungsleitung luft- und keimfrei mit der Zuleitung im Bereich zwischen Tropfkammer einerseits und Leukozytendepletionsfilter andererseits verbunden ist, da damit auf einfache Weise sichergestellt wird, daß das Blut bzw. seine Komponenten ohne Bildung von Ablagerungen durch die einschlägigen Leitungen vom Sammelbehälter zur Filteranordnung und von dort zu den Aufnahmebehältern für zelluläre Bestandteile bzw. Blutplasma fließt/fließen.

Als sehr vorteilhaft erweist sich außerdem eine Ausführungsform der in Rede stehenden Vorrichtung, bei der die vorgegebenen vertikalen Abstände mittels einer wiederverwendbaren und zumindest während der Blutaufnahme unveränderlichen mechanischen Haltevorrichtung mit definiert angeordneten Halteelementen für die Behälter und Filtereinrichtungen festlegbar sind, weil damit gewährleistet wird, daß die bei jedem einzelnen Einsatz einer Vorrichtung der vorliegenden Art erforderliche gegenseitige Anordnung von deren einzelnen Elementen ohne Schwierigkeiten in reproduzierbarer Weise herstellbar ist, insbesondere dann, wenn die einzelnen Halteelemente - beispielsweise starre Haken - in ausreichend deutlicher Weise gekennzeichet sind. Dabei kann die Haltevorrichtung selbst beispielsweise rahmen- oder baumartig ausgebildet, ortsfest angeordnet oder transportabel und starr oder zusammenlegbar gestaltet sein - sofern sie nur die Bedingung erfüllt, daß sie während einer Blutaufnahme in sich unveränderlich ist.

Ausführungsbeispiele der erfindungsgemäßen Filteranordnung und der zugehörigen Vorrichtung sind in der Zeichnung dargestellt.

Es zeigen:
- Fig. 1:: Senkrechter Schnitt durch eine erfindungsgemäße Filteranordnung in schematischer und teilweise durchbrochener Darstellung sowie in willkürlichem Maßstab.
- Fig. 2:: Schnitt A - A aus Fig. 1 in vergrößerter und vereinfachter schematischer Darstellung.
- Fig. 3:: Schnitt B - B aus Fig. 1 in vergrößerter und vereinfachter schematischer Darstellung.
- Fig. 4:: Ansicht einer Vorrichtung für den Einsatz der Filteranordnung gemäß Fig. 1 bis 3 am Spender in schematischer Darstellung.

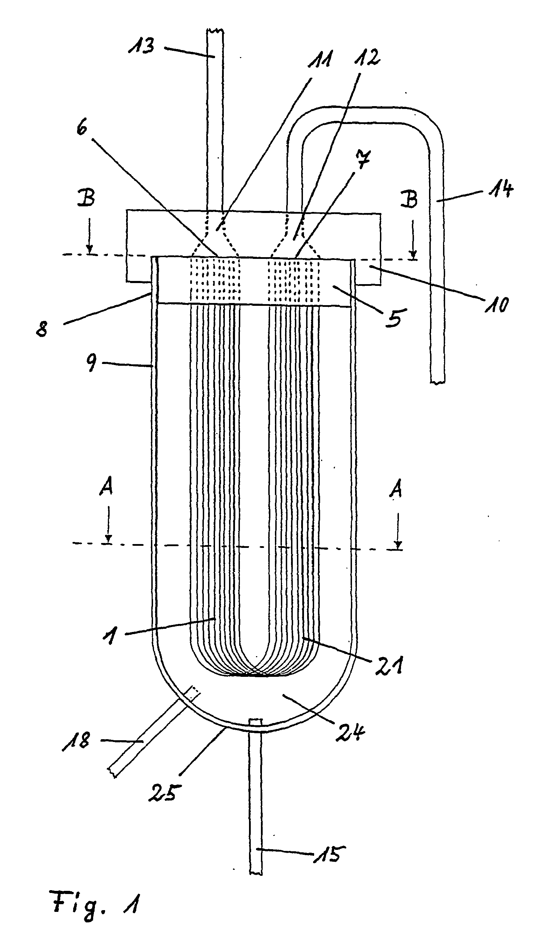

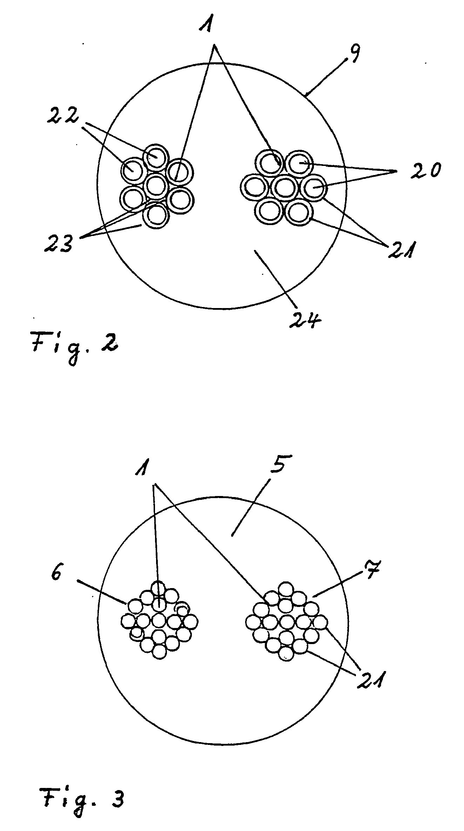

Fig. 1 zeigt einen senkrechten Schnitt durch eine erfindungsgemäße Filteranordnung in schematischer und teilweise durchbrochener Darstellung sowie in willkürlichem Maßstab, die ein U-förmig ausgebildetes Bündel 1 aus n = 650 hydrophilen mikroporösen Polyethersulfan - Kapillarhohlfasern 21 des Typs Micro PES-TF 10 der Akzo Faser AG mit einer jeweiligen freien Länge von 1 = 27 cm aufweist, deren Enden in einer gemeinsamen Lochplatte 5 luft- und keimdicht fixiert sind, wobei sie dort einen gemäß Fig. 3 deutlich erkennbaren Eingangsbereich 6 und einen ebenso deutlich erkennbaren Ausgangsbereich 7 ausbilden. Die freie Länge von 1 = 27 cm versteht sich als Länge jeder Hohlfaser 21 von der Unterseite der Lochplatte 5 bis zum erneuten Erreichen der Unterseite der Lochplatte 5. Die Gesamtlänge jeder Hohlfaser 21 ergibt sich durch doppelte Addition der Stärke der Lochplatte 5 zur freien Länge von 1 = 27 cm. Die Lochplatte 5 kann aus einem beliebigen geeigneten Material, beispielsweise Quarz, bestehen und eine vorgegebene Stärke von beispielsweise 3 cm aufweisen und ist im dargestellten Fall im oberen Endbereich 8 eines Gehäuses 9 luft- und keimdicht eingepaßt, das nur an einem seiner Enden, vorzugsweise am oberen, offen ist. Auf der Lochplatte 5 und dem oberen Randbereich 8 ist eine Verschlußkappe 10 angeordnet, die zwei Durchführungen 11, 12 aufweist, die zum einen den Eingangsbereich 6 und zum anderen den Ausgangsbereich 7 mit dem Außenraum des Gehäuses 9 verbinden. Ansonsten ist die Verschlußkappe 10 ebenfalls luft- und keimdicht mit der Lochplatte 5 und dem oberen Randbereich 8, vorzugsweise mittels Klebung, verbunden.

In die Durchführung 11 führt eine Zuleitung 13 für Spenderblut; aus der Durchführung 12 führt eine Ablaufleitung 14 der Einlaßkammer 22 (s. Fig. 2) heraus. Beide Leitungen 13, 14 sind luft- und keimdicht in den Durchführungen 11 bzw. 12 fixiert. An anderer Stelle des Gehäuses 9, vorzugsweise - aber nicht zwangsläufig - an seinem der Lochplatte 5 entgegengesetzten Ende, führt aus diesem eine luft- und keimdicht mit ihm verbundene Ablaufleitung 15 der Auslaßkammer 24 heraus, die vom Außenraum 23 (s. Fig. 2) aller Hohlfasern 21 im Gehäuse 9 unterhalb der Lochplatte 5 gebildet wird - genau so, wie die Einlaßkammer 22 von allen Innenräumen 20 (s. Fig. 2) der Hohlfasern 21 zwischen Eingangsbereich 6 und Ausgangsbereich 7 der Lochplatte 5 (s. Fig. 3) ausgebildet wird.

Der Transport des Blutes und seiner Komponenten erfolgt hier vorzugsweise durch die Schwerkraft, auch wenn es bisher schon verschiedene Entwicklungen gegeben hat, diesen Transport durch interne oder externe Vakuumerzeugung zu bewirken - was hier allerdings nicht ausgeschlossen sein soll.

Weiterhin zeigt die Fig. 1 eine Anschlußvorrichtung 18 bekannter Art für die Einleitung einer Kochsalzlösung zum Spülen der hydrophilen Hohlfasern 21 vor der Aufnahme von Spenderblut, die am Gehäuse 9 im Bereich der Auslaßkammer 24 angeordnet ist.

Die Fig. 2 zeigt den Schnitt A - A aus Fig. 1, der in vergrößerter und vereinfachter schematischer Darstellung den Aufbau des Bündels 1 aus den n = 650 Hohlfasern 21 der oben genannten Art offenbart, von denen hier zu Demonstrationszwecken nur einige wenige herangezogen wurden. Eine Mehrzahl von Hohlfasern 21 bildet aus der Gesamtheit ihrer Innenräume 20 die Einlaßkammer 22, die über die bereits mehrfach beschriebene Zuleitung 13 zum einen mit dem Spender und über die ebenfalls bereits mehrfach erwähnte Ablaufleitung 14 zum anderen mit einem Aufnahmebehälter für die zellulären Bestandteile des Blutes verbunden ist. Dagegen bildet der Außenraum 23 aller Hohlfasern 21 innerhalb des Gehäuses 9 die Auslaßkammer 24, die über die ebenfalls bereits mehrfach erwähnte Ablaufleitung 15 mit einem Aufnahmebehälter für das Plasma verbunden ist.

Die Fig. 3 zeigt den Schnitt B - B aus Fig. 1, der in ebenfalls vergrößerter und vereinfachter schematischer Darstellung die Aufsicht auf die Lochplatte 5 bei entfernter Verschlußkappe 10 und damit auf den Eingangsbereich 6 und den Ausgangsbereich 7 verdeutlicht.

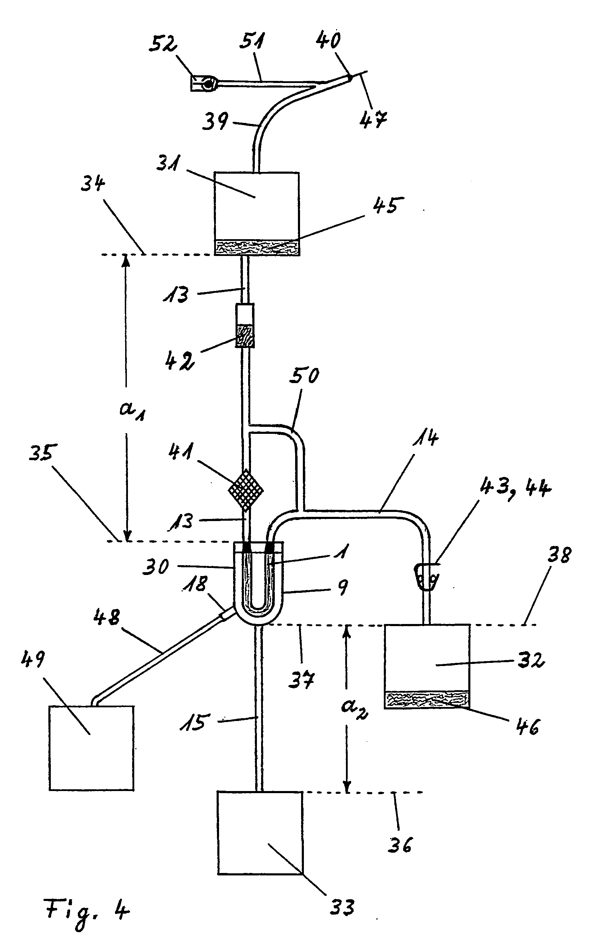

Die Fig. 4 zeigt die Ansicht einer Vorrichtung für den Einsatz der Filteranordnung gemäß Fig. 1 bis 3 am Spender in schematischer Darstellung, die zunächst aus einem hier - aber nicht zwangsläufig - als beutelartiger Hohlkörper aus Kunststoff-Folie bekannter Art vorausgesetzten Sammelbehälter 31 besteht, der luft- und keimdicht einerseits mit einer Eingangsleitung 39 und andererseits mit einer Zuleitung 13 zu einer Filteranordnung 30 verbunden ist, von denen - wie auch für alle weiteren noch zu beschreibenden Verbindungsleitungen - ohne Beschränkung der Allgemeinheit vorausgesetzt ist, daß sie schlauchartige Verbindungselemente aus elastischem Kunststoffmaterial bekannter Art sind. Die Eingangsleitung 39 trägt an ihrem dem Sammelbehälter 31 abgewandten Ende ein mit einem oder mehreren Blutgefäßen eines Spenders koppelbares Anschlußelement 40, von dem hier - aber nicht zwingend - angenommen ist, daß es sich dabei um eine Kanüle 47 herkömmlicher Art handelt. Letztere kann selbstverständlich auch durch jedes andere geeignete Element gleicher Wirkungsweise ersetzt werden.

Die Zuleitung 13 führt zunächst zu einer luft- und keimdicht in sie eingefügten Tropfkammer 42, die vorteilhafterweise zur Einschaltung und Kontrolle der der Filteranordnung 30 pro Zeiteinheit zugeführten Menge von Spenderblut dient und ihrerseits über die Fortsetzung der Zuleitung 13 mit einem Leukozytendepletionsfilter 41 bekannter Art verbunden ist, der ebenfalls luft- und keimdicht in die Zuleitung 13 eingefügt ist.

Die Anordnung des Leukozytendepletionsfilters 41 an der gezeigten Stelle ist nicht zwingend vorgegeben, weist jedoch den Vorteil auf, daß die nachgeschaltete Filteranordnung 30, die bereits vorstehend im Detail beschrieben worden ist, weniger belastet wird und daher eine schnellere Auftrennung zwischen Blutplasma und verbliebenen zellulären Bestandteilen des Blutes ermöglicht, wobei außerdem von vornherein eine häufig wünschenswerte Trennung der Leukozyten - und gleichzeitig der Thrombozyten - von den übrigen Komponenten einer zu trennenden Charge von Spenderblut erzielt wird. Darüber hinaus gewährleistet die dargestellte Anordnung des Leukozytendepletionsfilters 41 unmittelbar vor der Filteranordnung 30 einen ununterbrochenen gleichmä-ßigen Fluß des zu trennenden Spenderblutes durch beide vorgenannten Filtereinrichtungen 30, 41 und damit auch konstante vorgegebene Druckverhältnisse in diesen als eine der Voraussetzungen für den überraschenden Effekt, daß die in Rede stehende Vorrichtung ein vollkommen zellfreies Blutplasma in dem weiter unten noch zu erläuternden Aufnahmebehälter 33 für Blutplasma erzeugt.

Die Filteranordnung 30 besteht - wie bereits beschrieben - aus einem mit physiologischer Kochsalzlösung tränkbaren Bündel 1 von Hohlfasern 21, wobei die Gesamtheit der Innenräume 20 der Hohlfasern 21 eine Einlaßkammer 22 und der Außenraum 23 aller Hohlfasern 21 eine Auslaßkammer 24 ausbilden. Die Auslaßkammer 24 ist dann über eine luft- und keimdicht mit ihr verbundene Ablaufleitung 15 mit einem Aufnahmebehälter 33 für Blutplasma verbunden, der ebenfalls luft- und keimdicht an die Ablaufleitung 15 anschließt; die Einlaßkammer 22 ist ihrerseits über eine Ablaufleitung 14 mit einem Aufnahmebehälter 32 für zelluläre Bestandteile des Blutes verbunden, wobei selbstverständlich auch hier alle Verbindungsstellen luft- und keimdicht ausgeführt sind. Bei der in Rede stehenden Vorrichtung nimmt der Aufnahmebehälter 32 für zelluläre Bestandteile im wesentlichen sofort ein üblicherweise gewünschtes weitgehend reines Erythrozyten-Konzentrat auf. Bezüglich der vorstehend angeführten Bezugszeichen wird teilweise auf die Fig. 1 und 2 verwiesen, da die Verwendung aller dort definierten Bezugszeichen in der vorliegenden Fig. 4 deren Übersichtlichkeit erheblich beeinträchtigen würde.

Die optimale Funktionsweise der Filteranordnung 30 verlangt eine spezielle Einstellung der Druckverhältnisse im Gesamtsystem der vorliegenden Vorrichtung, insbesondere jedoch einen vorzugebenden Rückstau in der Filteranordnung 30, der dadurch hervorrufbar ist, daß zwischen den einzelnen Elementen der Vorrichtung, insbesondere zwischen dem Sammelbehälter 31 und der Filteranordnung 30 einerseits sowie der Filteranordnung 30 und den Aufnahmebehältern 32, 33 für zelluläre Bestandteile des Spenderblutes bzw. Blutplasma andererseits, vorgegebene vertikale Abstände eingehalten werden, was beispielsweise dadurch erreichbar ist, daß die einzelnen Elemente der Vorrichtung während jeder Blutaufnahme an speziell gekennzeichneten Halteelementen, beispielsweise starren Haken, einer zumindest während jeder Blutaufnahme unveränderlichen mechanischen Haltevorrichtung aufgehängt sind. Letztere ist vorteilhafterweise wiederverwendbar und kann darüber hinaus beispielsweise ortsfest angeordnet oder transportabel, rahmen- oder baumartig ausgebildet und starr oder zusammenlegbar gestaltet sein - sofern sie nur während jeder Blutaufnahme in sich unveränderlich ist und eine reproduzierbare Anordnung von Vorrichtungen der in Rede stehenden Art gestattet. Bei dem gezeigten Ausführungsbeispiel ergibt sich beispielsweise ein optimaler Hämatokritwert, wenn der Abstand zwischen der Unterkante 34 des Sammelbehälters 31 und der Oberkante 35 der Filteranordnung 30 a₁ = (100 ± 20) cm und der Abstand zwischen der Unterkante 37 der Filteranordnung 30 und der Oberkante 36 des Aufnahmebehälters 33 für Blutplasma a₂ = 50 cm beträgt, während die Oberkante 38 des Aufnahmebehälters 32 für zelluläre Bestandteile des Spenderblutes auf der Höhe der Unterkante 37 der Filteranordnung 30 angeordnet ist.

Der Abstand a₁ läßt sich zur Erzielung einer optimalen Trennung des Spenderblutes beispielsweise durch eine Anordnung verwirklichen, bei der zwischen Sammelbehälter 31 und Tropfkammer 32 ein Abstand von a₁₁ = 30 cm, zwischen Tropfkammer 42 und einem hier nicht explizit dargestellten Brechventil bekannter Art zum gezielten Starten des Blutflusses vom Sammelbehälter 31 zur Filteranordnung 30 ein Abstand von a₁₂ = 13 cm, zwischen Brechventil und dem Eingang einer weiter unten noch im Detail erläuterten Entlüftungsleitung 50 in die Zuleitung 13 ein Abstand von a₁₃ = 23 cm, zwischen Eingang der Entlüftungsleitung 50 und Leukozytendepletionsfilter 41 ein Abstand a₁₄ = 12 cm und zwischen Leukozytendepletionsfilter 41 und Filteranordnung 30 ein Abstand von a₁₅ = 12 cm gewählt wird, wobei zur Summe der vorgenannten Abstände a₁₁ bis a₁₅ noch die Gesamtlänge von Tropfkammer 42, Brechventil und Leukozytendepletionsfilter 41 hinzu zu addieren ist. Der Entlüftungsleitung 50 kann dabei eine Länge von 1_{E} = 30 cm zugeordnet werden, wobei deren Ausgang von der Ablaufleitung 14 in einem Abstand von a = 5 cm vom Austritt der Ablaufleitung 14 aus der Filteranordnung 30 angeordnet wird. Selbstverständlich kann diese Anordnung auch durch jede andere ersetzt werden, die bei sonst ungeänderten Parametern der vorliegenden Vorrichtung identische Druckverhältnisse in der Filteranordnung 30 erzeugt.

Zusätzlich zu der räumlich vorgegebenen Anordnung der vorbeschriebenen Elemente weist die gezeigte Vorrichtung noch eine weitere Einrichtung 43 zur Erzeugung eines Rückstaus in der Filteranordnung 30 auf, die beispielsweise durch eine an der Ablaufleitung 14 der Einlaßkammer 22 angeordnete einstellbare Klemmvorrichtung - hier in Form einer Rollklemme 44 bekannter Art - verwirklichbar ist und vorzugsweise - aber nicht nur - zur auf einfache Weise durchführbaren Korrektur und Nachjustierung von unvermeidlichen Schwankungen der Einstellungsparameter der Druckverhältnisse im Gesamtsystem der Vorrichtung, beispielsweise Viskositätsänderungen des Blutes von einem Spender zum anderen oder Temperaturschwankungen von einem Trennvorgang zum anderen, dient. Darüber hinaus zweigt von der Ablaufleitung 14 noch eine luft- und keimdicht mit ihr verbundene Entlüftungsleitung 50 ab, die ihrerseits luft- und keimdicht mit der Zuleitung 13 im Bereich zwischen Tropfkammer 42 und Leukozytendepletionsfilter 41 verbunden ist und bewirkt, daß das Blut bzw. seine Komponenten keine störenden Ablagerungen in den Leitungen 13, 14, 15 und den Filtereinrichtungen 30, 41 ausbilden.

Üblicherweise wird der Sammelbehälter 31 bereits vor der Sterilisation der gesamten Vorrichtung mit einer Stabilisatorflüssigkeit 45, beispielsweise CPD, befüllt; in gleicher Weise wird auch der Aufnahmebehälter 32 für zelluläre Bestandteile des Spenderblutes mit einer Additivlösung 46, beispielsweise SAG-Manitol, versehen. Beide Maßnahmen dienen der Sicherstellung einer ausreichenden Konservierungsdauer für die erzeugten Blutkomponenten.

Für die erforderliche Spülung der hydrophilen Hohlfasern 21 vor dem Trennvorgang mit einer physiologischen Kochsalzlösung ist die Filteranordnung 30 im übrigen über eine Anschlußvorrichtung 18 und eine Spülleitung 48 luft- und keimdicht mit einem Vorratsbehälter 49 für eine solche Kochsalzlösung verbunden.

Zur Entnahme von Spenderblut für Kontrolluntersuchungen weist die vorliegende Vorrichtung außerdem eine von der Eingangsleitung 39 im Bereich zwischen Anschlußelement 40 und Sammelbehälter 31, vorzugsweise - aber nicht zwangsläufig - in der Nähe des Anschlußelements 40, luft- und keimdicht abzweigende Prüfleitung 51 auf, deren der Eingangsleitung 39 abgewandtes Ende in einem durch äußere Druckerzeugung zu öffnenden Entnahmeventil 52 bekannter Art mündet.

Sämtliche luft- und keimdichten Verbindungen sind entweder Kleb- oder Schweißnähte oder Kopplungselemente bekannter Art, beispielsweise Steck- oder Schraubkupplungen, die hier - da dem Fachmann bekannt - nicht explizit dargestellt und beschrieben sind. Gleiches gilt für eventuell in den Leitungen 13, 14, 15, 50 anzubringende Einmalklemmen oder Brechventile, um eine Leitung, beispielsweise die Zuleitung 13, erst bei Bedarf an vorgegebenem Ort, beispielsweise zwischen Tropfkammer 42 und Eingang der Entlüftungsleitung 50, öffnen zu können oder im Notfall oder bei der Demontage der Vorrichtung nach dem Abschluß eines Trennvorgangs keine ungewollten Lufteinbrüche oder plötzlichen Druckänderungen in den mit den aufgetrennten Blutkomponenten gefüllten Behältern 32, 33 oder auch dem Vorratsbehälter 49 zu verursachen.

Selbstverständlich betrifft das vorliegende Patentbegehren nicht nur die vorstehend explizit erläuterten Ausführungsbeispiele, sondern alle denkbaren Ausführungsformen der der vorliegenden Patentanmeldung zugrundeliegenden erfinderischen Idee, die vom Schutzumfang der nachfolgenden Patentansprüche 1 und 12 erfaßt werden.

### Bezugszeichenliste

- 1: Bündel von Hohlfasern
- 5: Lochplatte
- 6: Eingangsbereich
- 7: Ausgangsbereich
- 8: offener Endbereich
- 9: Gehäuse
- 10: Verschlußkappe
- 11,12: Durchführungen
- 13: Zuleitung für Spenderblut
- 14: Ablaufleitung der Einlaßkammer
- 15: Ablaufleitung der Auslaßkammer
- 18: Anschlußvorrichtung für Kochsalzlösung
- 20: Innenräume
- 21: Hohlfasern
- 22: Einlaßkammer
- 23: Außenraum
- 24: Auslaßkammer
- 25: geschlossenes Ende
- 30: Filteranordnung
- 31: Sammelbehälter
- 32: Aufnahmebehälter für zelluläre Bestandteile des Spenderblutes
- 33: Aufnahmebehälter für Blutplasma
- 34: Unterkante des Sammelbehälters
- 35: Oberkante der Filteranordnung
- 36: Oberkante des Aufnahmebehälters für Blutplasma
- 37: Unterkante der Filteranordnung
- 38: Oberkante des Aufnahmebehälters für zelluläre Bestandteile des Spenderblutes
- 39: Eingangsleitung
- 40: Anschlußelement
- 41: Leukozytendepletionsfilter
- 42: Tropfkammer
- 43: Einrichtung zur Erzeugung eines Rückstaus
- 44: Rollklemme
- 45: Stabilisatorflüssigkeit
- 46: Additivlösung
- 47: Kanüle
- 48: Spülleitung
- 49: Vorratsbehälter für Kochsalzlösung
- 50: Entlüftungsleitung
- 51: Prüfleitung
- 52: Entnahmeventil

## Patentansprüche

1. Filteranordnung zum Auftrennen von Blut in Plasma und zelluläre Bestandteile mit einer Zuleitung (13) für das Spenderblut zur Einlaßkammer (22) mindestens eines mit einem geschlossenen Gehäuse (9) versehenen Filterelements, das mit einer Ablaufleitung (14) der Einlaßkammer zu mindestens einem externen Aufnahmebehälter (32) für die zellulären Bestandteile, mit einer innerhalb des Gehäuses von der Einlaßkammer durch eine feinporige Membran getrennten Auslaßkammer (24), mit einer Ablaufleitung (15) der Auslaßkammer zu mindestens einem externen Aufnahmebehälter (33) für Plasma und mit einer Einrichtung (43) zur Erzeugung eines Rückstaus im Filterelement versehen ist,
**dadurch gekennzeichnet,**
**daß** die feinporige Membran durch ein U-förmig ausgebildetes Bündel (1) von n = 650 hydrophilen mikroporösen Polyethersulfan - Kapillarhohlfasern (21) ausgebildet wird, deren Enden in einer gemeinsamen Lochplatte (5) mit einem Eingangs- (6) und einem Ausgangsbereich (7) luft- und keimdicht fixiert sind und deren freie Länge zwischen dem Eingangs- (6) und Ausgangsbereich (7) der Lochplatte (5) 1 = 27 cm beträgt, wobei die Lochplatte (5) ihrerseits luft- und keimdicht in den offenen Endbereich (8) eines nur einseitig offenen Gehäuses (9) so eingepaßt ist, daß die freie Länge aller Hohlfasern (21) innerhalb des durch die Lochplatte (5) abgeschlossenen Gehäuses (9) angeordnet ist und die Innenräume aller Hohlfasern (21) die Einlaßkammer (22) und der Außenraum (23) aller Hohlfasern (21) zwischen Lochplatte (5) und dem geschlossenen Ende (25) des Gehäuses (9) die Auslaßkammer (24) ausbilden.

2. Filteranordnung nach Anspruch 1,
**dadurch gekennzeichnet,**
**daß** das Gehäuse (9) an seinem offenen Endbereich (8) mit einer Verschlußkappe (10) versehen ist, die je eine Durchführung (11,12) zum Eingangs- (6) und zum Ausgangsbereich (7) der Lochplatte (5) freigibt und im übrigen luft- und keimdicht mit der Oberfläche der Lochplatte (5) und dem offenen Endbereich (8) des Gehäuses (9) verbunden ist.

3. Filteranordnung nach Anspruch 2,
**dadurch gekennzeichnet,**
**daß** die Durchführungen (11,12) luft- und keimdicht mit der Zuleitung (13) für das Spenderblut bzw. der Ablaufleitung (14) der Einlaßkammer (22) für die zellulären Bestandteile verbunden sind.

4. Filteranordnung nach Anspruch 2 oder 3,
**dadurch gekennzeichnet,**
**daß** die luft- und keimdichten Verbindungen zwischen Lochplatte (5) und Verschlußkappe (10) sowie zwischen den Durchführungen (11, 12) und der Zuleitung (13), für das Spenderblut bzw. der Ablaufleitung (14) der Einlaßkammer (22) für die zellulären Bestandteile Klebverbindungen sind.

5. Filteranordnung nach einem der Ansprüche 1 bis 4,
**dadurch gekennzeichnet,**
**daß** die Lochplatte (5) aus Quarz besteht und die Hohlfasern (21) in dieser Lochplatte (5) mittels Verfahren bekannter Art fixiert sind.

6. Filteranordnung nach Anspruch 5,
**dadurch gekennzeichnet,**
**daß** die Lochplatte (5) eine Stärke von 3 cm aufweist und die Gesamtlänge jeder Hohlfaser (21) demzufolge l_{g} = 33 cm beträgt.

7. Filteranordnung nach einem der Ansprüche 1 bis 6,
**dadurch gekennzeichnet,**
**daß** die Ablaufleitung (15) der Auslaßkammer (24) am geschlossenen Ende (25) des Gehäuses (9) angeordnet ist.

8. Filteranordnung nach einem der Ansprüche 1 bis 7,
**dadurch gekennzeichnet,**
**daß** das Gehäuse (9) aus Glas besteht.

9. Filteranordnung nach einem der Ansprüche 1 bis 7,
**dadurch gekennzeichnet,**
**daß** das Gehäuse (9) aus einem Kunststoff besteht.

10. Filteranordnung nach Anspruch 9,
**dadurch gekennzeichnet,**
**daß** der Kunststoff Polyurethan ist.

11. Filteranordnung nach einem der Ansprüche 1 bis 10,
**dadurch gekennzeichnet,**
**daß** das Gehäuse (9) im Bereich der Auslaßkammer (24) mit einer Anschlußvorrichtung (18) bekannter Art für die Einleitung einer Kochsalzlösung zum Spülen der hydrophilen Hohlfasern (21) vor der Aufnahme von Spenderblut versehen ist.

12. Vorrichtung mit der Filteranordnung nach einem der Ansprüche 1 bis 11 für den Einsatz am Spender,
**dadurch gekennzeichnet,**
**daß** dem Eingangsbereich (6) der Filteranordnung (30) ein Sammelbehälter (31) für das Spenderblut vor-, dem Auslaßbereich (7) der Filteranordnung (30) ein Aufnahmebehälter (32) für zelluläre Bestandteile des Spenderblutes nach- und der Auslaßkammer (24) der Filteranordnung (30) ein Aufnahmebehälter (33) für Blutplasma ebenfalls nachgeschaltet ist, wobei die Verbindungen zwischen der Filteranordnung (30) und den vorgenannten Behältern (31, 32, 33) über die Zuleitung (13), die Ablaufleitung (14) und die Ablaufleitung (15) luft- und keimdicht, aber mit Mitteln bekannter Art ohne Luftzutritt und Störeffekte durch unerwünschte Druckausgleichseffekte unterbrechbar ausgebildet sind,
**daß** der Sammelbehälter (31) mit einem bestimmten vertikalen Abstand seiner Unterkante (34) oberhalb der Oberkante (35) der Filteranordnung (30), der Aufnahmebehälter (33) für Blutplasma mit einem bestimmten vertikalen Abstand seiner Oberkante (36) unterhalb der Unterkante (37) der Filteranordnung (30) und der Aufnahmebehälter (32) für zelluläre Bestandteile des Spenderblutes mit seiner Oberkante (38) auf der Höhe der Unterkante (37) der Filteranordnung (30) angeordnet ist und
**daß** der Sammelbehälter (31) mit einer luft- und keimdicht mit ihm verbundenen Eingangsleitung (39) verbunden ist, die an ihrem dem Sammelbehälter (31) abgewandten Ende mit einem Anschlußelement (40) zum Blutkreislauf des Spenders versehen ist.

13. Vorrichtung nach Anspruch 12,
**dadurch gekennzeichnet,**
**daß** in der Zuleitung (13) zwischen Sammelbehälter (31) und Filteranordnung (30) ein Leukozytendepletionsfilter (41) bekannter Art angeordnet ist.

14. Vorrichtung nach Anspruch 12 oder 13,
**dadurch gekennzeichnet,**
**daß** in der Zuleitung (13) zwischen Sammelbehälter (31) und Filteranordnung (30) eine Tropfkammer (42) bekannter Art angeordnet ist.

15. Vorrichtung nach Anspruch 14
**dadurch gekennzeichnet,**
**daß** die Tropfkammer (42) zwischen Sammelbehälter (31) und Leukozytendepletionsfilter (41) angeordnet ist.

16. Vorrichtung nach einem der Ansprüche 12 bis 15,
**dadurch gekennzeichnet,**
**daß** der vorgegebene vertikale Abstand zwischen der Unterkante (34) des Sammelbehälters (31) und der Oberkante (35) der Filteranordnung (30) a₁ = (100 ± 20) cm beträgt.

17. Vorrichtung nach einem der Ansprüche 12 bis 16,
**dadurch gekennzeichnet,**
**daß** der vorgegebene vertikale Abstand zwischen der Unterkante (37) der Filteranordnung (30) und der Oberkante (36) des Aufnahmebehälters (33) für Blutplasma a₂ = 50 cm beträgt.

18. Vorrichtung nach einem der Ansprüche 12 bis 17,
**dadurch gekennzeichnet,**
**daß** die Ablaufleitung (14) der Einlaßkammer (22) mit einer Einrichtung zur Erzeugung eines Rückstaus (43) in der Filteranordnung (30) versehen ist.

19. Vorrichtung nach Anspruch 18,
**dadurch gekennzeichnet,**
**daß** die Einrichtung zur Erzeugung eines Rückstaus (43) eine einstellbare Klemmvorrichtung ist.

20. Vorrichtung nach Anspruch 19,
**dadurch gekennzeichnet,**
**daß** die einstellbare Klemmvorrichtung durch eine Rollklemme (44) bekannter Art verwirklicht wird.

21. Vorrichtung nach einem der Ansprüche 12 bis 20,
**dadurch gekennzeichnet,**
**daß** der Sammelbehälter (32) mindestens im Startzeitpunkt der Blutaufnahme eine bestimmte Menge einer Stabilisatorflüssigkeit (45) bekannter Art enthält.

22. Vorrichtung nach einem der Ansprüche 12 bis 21,
**dadurch gekennzeichnet,**
**daß** der Aufnahmebehälter (32) für zelluläre Bestandteile des Spenderblutes eine Vorfüllung mit einer bestimmten Menge einer Additivlösung (46) bekannter Art aufweist.

23. Vorrichtung nach einem der Ansprüche 12 bis 22,
**dadurch gekennzeichnet,**
**daß** das Anschlußelement (40) eine Kanüle (47) ist.

24. Vorrichtung nach einem der Ansprüche 12 bis 23,
**dadurch gekennzeichnet,**
**daß** die Auslaßkammer (24) über die Anschlußvorrichtung (18) für Kochsalzlösung luft- und keimfrei mit einem Vorratsbehälter (49) für eine solche Kochsalzlösung verbunden ist.

25. Vorrichtung nach einem der Ansprüche 13 bis 24,
**dadurch gekennzeichnet,**
**daß** die Ablaufleitung (14) der Einlaßkammer (22) mittels einer Entlüftungsleitung (50) luft- und keimfrei mit der Zuleitung (13) im Bereich zwischen Tropfkammer (42) einerseits und Leukozytendepletionsfilter (41) andererseits verbunden ist.

26. Vorrichtung nach einem der Ansprüche 12 bis 25,
**dadurch gekennzeichnet,**
**daß** die bestimmten vertikalen Abstände mittels einer wiederverwendbaren und zumindest während der Blutaufnahme unveränderlichen mechanischen Haltevorrichtung mit definiert angeordneten Halteelementen für die Behälter (31, 32, 33, 49) und Filtereinrichtungen (30, 41) festlegbar sind.

## Claims

1. Filter arrangement for the separation of blood into plasma and cellular components, with an admission line (13) for delivering the donor blood to the inlet chamber (22) of at least one filter element which is provided with a closed housing (9) and which is provided with a discharge line (14) leading from the inlet chamber to at least one external collection container (32) for the cellular components, with an outlet chamber (24) separated from the inlet chamber, within the housing, by a finely porous membrane, with a discharge line (15) leading from the outlet chamber to at least one external collection container (33) for plasma, and with a device (43) for generating a back-pressure in the filter element, **characterized in that** the finely porous membrane is formed by a U-shaped bundle (1) of n = 650 hydrophilic microporous polyethersulphone capillary hollow fibres (21) whose ends are fixed in an air-tight and germ-proof manner in a common perforated plate (5) with an inlet area (6) and an outlet area (7), and whose free length between the inlet area (6) and outlet area (7) of the perforated plate (5) is 1 = 27 cm, the perforated plate (5) itself being fitted in an air-tight and germ-proof manner into the open end area (8) of a housing (9), which is open only at one end, in such a way that the free length of all the hollow fibres (21) is arranged inside the housing (9) closed off by the perforated plate (5), and the inlet chamber (22) is formed by the inner volumes of all the hollow fibres (21), and the outlet chamber (24) is formed by the external volume (23) of all the hollow fibres (21) between the perforated plate (5) and the closed end (25) of the housing (9).

2. Filter arrangement according to Claim 1, **characterized in that**, at its open end area (8), the housing (9) is provided with a closure cap (10) which leaves a passage (11, 12) to the inlet area (6) and outlet area (7), respectively, of the perforated plate (5) and is also connected in an air-tight and germ-proof manner to the surface of the perforated plate (5) and to the open end area (8) of the housing (9).

3. Filter arrangement according to Claim 2, **characterized in that** the passages (11, 12) are connected in an air-tight and germ-proof manner to the admission line (13) for the donor blood and, respectively, to the discharge line (14) of the inlet chamber (22) for the cellular components.

4. Filter arrangement according to Claim 2 or 3, **characterized in that** the air-tight and germ-proof connections between perforated plate (5) and closure cap (10), and also between the passages (11, 12) and the admission line (13) for the donor blood and, respectively, the discharge line (14) of the inlet chamber (22) for the cellular components, are adhesive bonded connections.

5. Filter arrangement according to one of Claims 1 to 4, **characterized in that** the perforated plate (5) is made of quartz, and the hollow fibres (21) are fixed in this perforated plate (5) by methods of known type.

6. Filter arrangement according to Claim 5, **characterized in that** the perforated plate (5) has a thickness of 3 cm, and the total length of each hollow fibre (21) accordingly measures l_{g} = 33 cm.

7. Filter arrangement according to one of Claims 1 to 6, **characterized in that** the discharge line (15) of the outlet chamber (24) is arranged at the closed end (25) of the housing (9).

8. Filter arrangement according to one of Claims 1 to 7, **characterized in that** the housing (9) is made of glass.

9. Filter arrangement according to one of Claims 1 to 7, **characterized in that** the housing (9) is made of a plastic.

10. Filter arrangement according to Claim 9, **characterized in that** the plastic is polyurethane.

11. Filter arrangement according to one of Claims 1 to 10, **characterized in that**, in the area of the outlet chamber (24), the housing (9) is provided with a connector device (18) of a known type for introduction of a saline solution for flushing the hydrophilic hollow fibres (21) before donor blood is received.

12. Device with the filter arrangement according to one of Claims 1 to 11, for application on the donor, **characterized in that** a collection container (31) for donor blood is connected upstream of the inlet area (6) of the filter arrangement (30), a collection container (32) for cellular components of the donor blood is connected downstream of the outlet area (7) of the filter arrangement (30), and a collection container (33) for blood plasma is likewise connected downstream of the outlet chamber (24) of the filter arrangement (30), the connections between the filter arrangement (30) and the aforementioned containers (31, 32, 33) via the admission line (13), the discharge line (14) and the discharge line (15) being made air-tight and germ-proof, but being able to be interrupted by means of a known type without intake of air and without interference caused by undesired pressure compensation effects, **in that** the collection container (31) is arranged with its bottom edge (34) at a defined vertical distance above the top edge (35) of the filter arrangement (30), the collection container (33) for blood plasma is arranged with its top edge (36) at a defined vertical distance below the bottom edge (37) of the filter arrangement (30), and the collection container (32) for cellular components of the donor blood is arranged with its top edge (38) level with the bottom edge (37) of the filter arrangement (30), and **in that** the collection container (31) is connected to an inlet line (39) which is connected to it in an air-tight and germ-proof manner and which, at its end remote from the collection container (31), is provided with a connector element (40) for connection to the blood circulation of the donor.

13. Device according to Claim 12, **characterized in that** a leukocyte depletion filter (41) of a known type is arranged in the admission line (13) between collection container (31) and filter arrangement (30).

14. Device according to Claim 12 or 13, **characterized in that** a drip chamber (42) of a known type is arranged in the admission line (13) between collection container (31) and filter arrangement (30).

15. Device according to Claim 14, **characterized in that** the drip chamber (42) is arranged between collection container (31) and leukocyte depletion filter (41).

16. Device according to one of Claims 12 to 15, **characterized in that** the defined vertical distance between the bottom edge (34) of the collection container (31) and the top edge (35) of the filter arrangement (30) is a₁ = (100 ± 20) cm.

17. Device according to one of Claims 12 to 16, **characterized in that** the defined vertical distance between the bottom edge (37) of the filter arrangement (30) and the top edge (36) of the collection container (33) for blood plasma is a₂ = 50 cm.

18. Device according to one of Claims 12 to 17, **characterized in that** the discharge line (14) of the inlet chamber (22) is provided with a device (43) for generating a back-pressure in the filter arrangement (30).

19. Device according to Claim 18, **characterized in that** the device (43) for generating a back-pressure is an adjustable clamping device.

20. Device according to Claim 19, **characterized in that** the adjustable clamping device is in the form of a roller clamp (44) of a known type.

21. Device according to one of Claims 12 to 20, **characterized in that**, at least at the time the blood withdrawal starts, the collection container (32) contains a defined amount of a stabilizer fluid (45) of a known type.

22. Device according to one of Claims 12 to 21, **characterized in that** the collection container (32) for cellular components of the donor blood is prefilled with a defined amount of an additive solution (46) of a known type.

23. Device according to one of Claims 12 to 22, **characterized in that** the connector element (40) is a cannula (47).

24. Device according to one of Claims 12 to 23, **characterized in that** the outlet chamber (24) is connected in an air-tight and germ-proof manner, via the connector device (18) for saline solution, to a supply reservoir (49) for such a saline solution.

25. Device according to one of Claims 13 to 24, **characterized in that** the discharge line (14) of the inlet chamber (22) is connected in an air-tight and germ-proof manner, by means of a venting line (50), to the admission line (13) in the area between the drip chamber (42), on the one hand, and the leukocyte depletion filter (41), on the other.

26. Device according to one of Claims 12 to 25, **characterized in that** the defined vertical distances can be fixed by means of a reusable mechanical holding device which is unchangeable at least during the blood withdrawal and which comprises holding elements arranged in a defined manner for the containers (31, 32, 33, 49) and the filter means (30, 41).

## Revendications

1. Agencement de filtre pour fractionner du sang en plasma et en composants cellulaires comprenant une conduite d'alimentation (13) pour le sang du donneur à la chambre d'entrée (22) d'au moins un élément de filtre pourvu d'un boîtier fermé (9) qui est pourvu d'une conduite de sortie (14) de la chambre d'entrée allant à au moins un récipient de stockage externe (32) pour les composants cellulaires, d'une chambre de sortie (24) à l'intérieure du boîtier, séparée de la chambre d'entrée par une membrane à pores fins, d'une conduite de sortie (15) de la chambre de sortie allant à au moins un récipient de stockage externe (33) pour le plasma et d'un dispositif (43) pour produire un non-retour dans l'élément de filtre,
**caractérisé en ce que**
la membrane à pores fins est réalisée par un faisceau (1) en forme de U de n = 650 fibres creuses capillaires hydrophiles microporeuses en polyéthersulfate (21), dont les extrémités sont fixées de manière étanche à l'air et aux germes dans une plaque perforée commune (5) avec une zone d'entrée (6) et une zone de sortie (7) et dont la longueur libre entre la zone d'entrée (6) et la zone de sortie (7) de la plaque perforée (5) vaut 1 = 27 cm, la plaque perforée (5) étant pour sa part adaptée de manière étanche à l'air et aux germes dans la zone d'extrémité ouverte (8) d'un boîtier (9) ouvert uniquement d'un côté, de telle sorte que la longueur libre de toutes les fibres creuses (21) soit disposée à l'intérieur du boîtier (9) fermé par la plaque perforée (5) et que les espaces internes de toutes les fibres creuses (21) constituent la chambre d'entrée (22) et que l'espace de sortie (23) de toutes les fibres creuses (21) entre la plaque perforée (5) et l'extrémité fermée (25) du boîtier (9) constitue la chambre de sortie (24).

2. Agencement de filtre selon la revendication 1,
**caractérisé en ce que**
le boîtier (9) est pourvu dans sa zone d'extrémité ouverte (8) d'un capuchon de fermeture (10) qui libère un passage respectif (11, 12) pour la zone d'entrée (6) et pour la zone de sortie (7) de la plaque perforée (5) et qui est connecté du reste de manière étanche à l'air et aux germes à la surface de la plaque perforée (5) et à la zone d'extrémité ouverte (8) du boîtier (9).

3. Agencement de filtre selon la revendication 2,
**caractérisé en ce que**
les passages (11, 12) sont connectés de manière étanche à l'air et aux germes à la conduite d'alimentation (13) pour le sang du donneur, et respectivement à la conduite de sortie (14) de la chambre d'entrée (22) pour les composants cellulaires.

4. Agencement de filtre selon la revendication 2 ou 3, **caractérisé en ce que**
les connexions étanches à l'air et aux germes entre la plaque perforée (5) et le capuchon de fermeture (10) ainsi qu'entre les passages (11, 12) et la conduite d'alimentation (13) pour le sang du donneur ou la conduite de sortie (14) de la chambre d'entrée (22) pour les composants cellulaires sont des connexions adhésives.

5. Agencement de filtre selon l'une quelconque des revendications 1 à 4,
**caractérisé en ce que**
la plaque perforée (5) est en quartz et les fibres creuses (21) sont fixées dans cette plaque perforée (5) au moyen de procédés connus.

6. Agencement de filtre selon la revendication 5,
**caractérisé en ce que**
la plaque perforée (5) a une épaisseur de 3 cm et la longueur totale de chaque fibre creuse (21) vaut par conséquent l_{g} = 33 cm.

7. Agencement de filtre selon l'une quelconque des revendications 1 à 6,
**caractérisé en ce que**
la conduite de sortie (15) de la chambre de sortie (24) est disposée à l'extrémité fermée (25) du boîtier (9).

8. Agencement de filtre selon l'une quelconque des revendications 1 à 7,
**caractérisé en ce que**
le boîtier (9) est en verre.

9. Agencement de filtre selon l'une quelconque des revendications 1 à 7,
**caractérisé en ce que**
le boîtier (9) est en plastique.

10. Agencement de filtre selon la revendication 9,
**caractérisé en ce que**
le plastique est du polyuréthanne.

11. Agencement de filtre selon l'une quelconque des revendications 1 à 10,
**caractérisé en ce que**
le boîtier (9) est pourvu dans la zone de la chambre de sortie (24) d'un dispositif de raccordement (18) de type connu pour l'introduction d'une solution saline pour rincer les fibres creuses hydrophiles (21) avant de recevoir le sang du donneur.

12. Dispositif comprenant l'agencement de filtre selon l'une quelconque des revendications 1 à 11, pour l'utilisation sur un donneur,
**caractérisé en ce que**
l'on monte avant la zone d'entrée (6) de l'agencement de filtre (30) un récipient collecteur (31) pour le sang du donneur, après la zone de sortie (7) de l'agencement de filtre (30) un récipient de stockage (32) pour les composants cellulaires du sang du donneur, et également après la chambre de sortie (24) de l'agencement de filtre (30) un récipient de stockage (33) pour le plasma sanguin, les connexions entre l'agencement de filtre (30) et les récipients mentionnés (31, 32, 33) étant réalisées par le biais de la conduite d'alimentation (13), de la conduite de sortie (14) et de la conduite de sortie (15) de manière étanche à l'air et aux germes, mais de manière à ce qu'elles puissent être interrompues avec des moyens de type connu sans entrée d'air et sans effets délétères dus à des effets de compensation de pression indésirables,
**en ce que** le récipient collecteur (31) est disposé avec une certaine distance verticale de son bord inférieur (34) au-dessus du bord supérieur (35) de l'agencement de filtre (30), le récipient de stockage (33) pour le plasma sanguin est disposé avec une certaine distance verticale de son bord supérieur (36) en dessous du bord inférieur (37) de l'agencement de filtre (30) et le récipient de stockage (32) pour les composants cellulaires du sang du donneur est disposé avec son bord supérieur (38) à la hauteur du bord inférieur (37) de l'agencement de filtre (30) et
**en ce que** le récipient collecteur (31) est connecté à une conduite d'entrée (39) connectée à lui de manière étanche à l'air et aux germes, qui est pourvue à son extrémité opposée au récipient collecteur (31) d'un élément de raccordement (40) pour le circuit sanguin du donneur.

13. Dispositif selon la revendication 12,
**caractérisé en ce que**
l'on dispose dans la conduite d'alimentation (13) entre le récipient collecteur (31) et l'agencement de filtre (30) un filtre de déplétion en leucocytes (41) de type connu.

14. Dispositif selon la revendication 12 ou 13,
**caractérisé en ce que**
l'on dispose dans la conduite d'alimentation (13) entre le récipient collecteur (31) et l'agencement de filtre (30) une chambre de goutte-à-goutte (42) de type connu.

15. Dispositif selon la revendication 14,
**caractérisé en ce que**
la chambre de goutte-à-goutte (42) est disposée entre le récipient collecteur (31) et le filtre de déplétion en leucocytes (41).

16. Dispositif selon l'une quelconque des revendications 12 à 15,
**caractérisé en ce que**
la distance verticale prédéterminée entre le bord inférieur (34) du récipient collecteur (31) et le bord supérieur (35) de l'agencement de filtre (30) vaut a₁ = (100 ± 20) cm.

17. Dispositif selon l'une quelconque des revendications 12 à 16,
**caractérisé en ce que**
la distance verticale prédéterminée entre le bord inférieur (37) de l'agencement de filtre (30) et le bord supérieur (36) du récipient de stockage (33) pour le plasma sanguin vaut a₂ = 50 cm.

18. Dispositif selon l'une quelconque des revendications 12 à 17,
**caractérisé en ce que**
la conduite de sortie (14) de la chambre d'entrée (22) est pourvue d'un dispositif pour produire un non-retour (43) dans l'agencement de filtre (30).

19. Dispositif selon la revendication 18,
**caractérisé en ce que**
le dispositif pour produire un non-retour (43) est un dispositif de serrage ajustable.

20. Dispositif selon la revendication 19,
**caractérisé en ce que**
le dispositif de serrage ajustable est mis en oeuvre par une pince roulante (44) de type connu.

21. Dispositif selon l'une quelconque des revendications 12 à 20,
**caractérisé en ce que**
le récipient collecteur (32) contient au moins à l'instant du début du prélèvement du sang une certaine quantité de liquide stabilisateur (45) de type connu.

22. Dispositif selon l'une quelconque des revendications 12 à 21,
**caractérisé en ce que**
le récipient de stockage (32) pour les composants cellulaires du sang du donneur présente un préremplissage avec une certaine quantité de solution additive (46) de type connu.

23. Dispositif selon l'une quelconque des revendications 12 à 22,
**caractérisé en ce que**
l'élément de raccordement (40) est une canule (47).

24. Dispositif selon l'une quelconque des revendications 12 à 23,
**caractérisé en ce que**
la chambre de sortie (24) est connectée par le biais du dispositif de raccordement (18) pour une solution saline de manière étanche à l'air et aux germes à un réservoir (49) pour une telle solution saline.

25. Dispositif selon l'une quelconque des revendications 13 à 24,
**caractérisé en ce que**
la conduite de sortie (14) de la chambre d'entrée (22) est connectée au moyen d'une conduite de désaérage (50) de manière étanche à l'air et aux germes à la conduite d'alimentation (13) dans la zone entre la chambre de goutte-à-goutte (42) d'une part et le filtre de déplétion en leucocytes (41) d'autre part.

26. Dispositif selon l'une quelconque des revendications 12 à 25,
**caractérisé en ce que**
les distances verticales déterminées peuvent être établies au moyen d'un dispositif de maintien mécanique réutilisable et au moins invariable pendant le prélèvement du sang, avec des éléments de maintien disposés de manière définie pour les récipients (31, 32, 33, 49) et les dispositifs de filtre (30, 41).
